# EUROPÄISCHE PATENTSCHRIFT

(11) **EP 2 989 978 B1**
(45) Veröffentlichungstag und Bekanntmachung des Hinweises auf die Patenterteilung: **08.03.2017**
(21) Anmeldenummer: 15002066.7
(22) Anmeldetag: 10.07.2015
(51) Int. Cl.: A61B 5/08

(54) **BEATMUNGSGERÄT ZUR DETEKTION EINER CHEYENE-STROKES-ATMUNG**
ARTIFICIAL RESPIRATION DEVICE FOR DETECTION OF CHEYENE-STROKES-RESPIRATION
APPAREIL DE RESPIRATION POUR DETECTION DE UNE CHEYENE-STROKES-RESPIRATION

(30) Priorität: 28.08.2014 DE 102014012791
(43) Veröffentlichungstag der Anmeldung: 02.03.2016
(73) Patentinhaber: Löwenstein Medical Technology S.A., 2557 Luxembourg (LU)
(72) Erfinder: Dömer, Benno, 76275 Ettlingen (DE); Klausmann, Dominic, 76106 Freiburg (DE)
(74) Vertreter: Marx, Thomas

(56) Entgegenhaltungen:
- WO-A1-2013/110136
- US-A1- 2006 070 624
- US-A1- 2008 183 083
- US-B1- 7 070 568

## Beschreibung

Die vorliegende Erfindung betrifft ein Beatmungsgerät mit wenigstens einer Beatmungseinrichtung zu Erzeugung eines Luftstroms für die Beatmung und mit wenigstens einer Überwachungseinrichtung. Die Überwachungseinrichtung dient zur Erfassung wenigstens eines Beatmungsparameters und zur Typisierung von Ereignissen in der Atmung anhand einer Überwachung des Beatmungsparameters.

Bei Beatmungsgeräten ist es oft von Vorteil, wenn die Möglichkeit einer Erkennung von Atemstörungen während der Beatmung besteht. Das ermöglicht einerseits eine Kontrolle der Effizienz der Beatmung und andererseits können Grund- und Nebenerkrankungen besser erkannt werden.

Eine Form einer solchen Atemstörung ist die sogenannte Cheyne-Stokes-Atmung (CS-Atmung). Die CS-Atmung wird häufig durch eine fehlerhafte Atemregelung hervorgerufen. Auslöser bzw. Grunderkrankung ist dabei häufig die Herzinsuffizienz. Die CS-Atmung ist gekennzeichnet durch abwechselnde, sanft ineinander übergehende Phasen von Hypoventilation(Hypopnoen und Apnoen) und Hyperventilationen. An die flachsten Atemzüge kann sich häufig auch ein kürzerer Atemstillstand anschließen. Danach vertiefen sich die Atemzüge zunehmend wieder.

Im Stand der Technik sind eine Reihe von Verfahren und Vorrichtungen bekannt geworden, welche eine Erkennung der CS-Atmung während einer Beatmung ermöglichen. Beispielsweise beschreibt die EP 1 867 227 A2 eine CS-Erkennung anhand eines Peaks im Spektrogramm, dem Flow und einer Apnoe-/ Hypopnoe-Erkennung. Zudem wird das Maß für die Steilheit des Wiederanstiegs der Atmung nach der Hypoventilation herangezogen. Die EP 2 421 435 A1 erkennt die CS-Atmung anhand eines Oxymetersignals über die Länge und den Wiederanstieg der Aufsättigung. Die WO 2013 110 136 erkennt die CS-Atmung anhand eines Histogramms der Zykluslängen von Apnoen und Hypopnoen, welche im Zuge einer Eventerkennung herangezogen werden. Die CS-Erkennung bei der WO 2009 118 737 erfolgt mittels einer Bestimmung des Anfangs und Endes von Apnoen, einer Berechnung von Zeitversätzen zwischen den Apnoen und einer anschließenden Generierung einer Ausgabe auf Basis des Vielfachen der Zykluslängen. Die US 2008 183 083 beschreibt eine Registrierung von aufeinanderfolgenden Pulslaufzeiten (PTT) des Patienten, mit Hilfe eines Pulsoximeters. Durch Analyse des resultierenden PTT-Signals wird ein Vorhandensein oder Fehlen von zentraler Schlafapnoe (CSA) oder obstruktiver Schlafapnoe (OSA) erfasst, um darüber Rückschlüsse auf eine Herzinsuffizienz zu ermöglichen, um basierend auf diesen Informationen die Funktion eines Herzschrittmachers zu steuern.

Die Aufgabe der vorliegenden Erfindung ist es, ein Beatmungsgerät zur Verfügung zu stellen, welches eine alternative, zuverlässige und unaufwendige Erkennung einer CS-Atmung ermöglicht.

Diese Aufgabe wird gelöst durch ein Beatmungsgerät mit den Merkmalen des Anspruchs 1. Einige Vorteile und Merkmale der vorliegenden Erfindung ergeben sich aus den Unteransprüchen weitere Vorteile und Merkmale sind in der allgemeinen Beschreibung und der Beschreibung des Ausführungsbeispiels angegeben.

Das erfindungsgemäße Beatmungsgerät umfasst wenigstens eine Beatmungseinrichtung zur Erzeugung eines Luftstromes für die Beatmung und wenigstens eine Überwachungseinrichtung. Die Überwachungseinrichtung dient zur Erfassung wenigstens eines Beatmungsparameters und zur Typisierung von Ereignissen in der Atmung anhand einer Überwachung des Beatmungsparameters. Dabei ist die Überwachungseinrichtung dazu geeignet und ausgebildet, wenigstens eine Ereignisanalyse zur Erkennung zeitlich aufeinander folgender Ereignisse durchzuführen. Die Ereignisanalyse dient dabei zur Erkennung eines Auftretens der Ereignisse, welches charakteristisch für eine CS-Atmung ist. Für die Ereignisanalyse ist die Überwachungseinrichtung dazu geeignet und ausgebildet, Periodenlängen zwischen den Ereignissen zu ermitteln. Zudem ist die Überwachungseinrichtung dazu geeignet und ausgebildet, die ermittelten Periodenlängen wenigstens teilweise miteinander zu vergleichen. Die Überwachungseinrichtung ist zudem dazu geeignet und ausgebildet, das Vorliegen einer CS-Atmung zu registrieren, wenn der Vergleich der Periodenlängen ergibt, dass die verglichenen Periodenlängen um weniger als 40% voneinander abweichen.

Das erfindungsgemäße Beatmungsgerät hat viele Vorteile. Ein erheblicher Vorteil ist, dass eine Überwachungseinrichtung vorgesehen ist, mit der ein charakteristisches Auftreten von zeitlich aufeinander folgenden Ereignissen zur Erkennung einer CS-Atmung herangezogen wird. Die Überwachungseinrichtung identifiziert das charakteristische Auftreten dabei anhand eines Vergleichs der Periodenlängen zwischen den Ereignissen und einer entsprechenden Bewertung der Abweichung bestimmter Periodenlängen voneinander. Eine derartige Erkennung der CS-Atmung ist sehr zuverlässig, da die Periodizität bestimmter Ereignisse ein wesentliches Merkmal einer CS-Atmung ist.

Zudem kann eine solche Überwachungseinrichtung, die zur Erkennung einer CS-Atmung eine Ereignisanalyse durchführt, vergleichsweise unaufwendig verwirklicht werden. Viele Geräte verfügen bereits über die Möglichkeit, Ereignisse in der Atmung zu erfassen und als bestimmte Typen zu erkennen. Die typisierten Ereignisse können dann als Grundlage für die CS-Erkennung herangezogen werden.

Insbesondere wird ein charakteristisches Auftreten von wenigstens zwei und vorzugsweise drei zeitlich aufeinander folgenden Ereignissen zur Erkennung einer CS-Atmung herangezogen. Vorzugsweise werden die Periodenlängen benachbarter Ereignisse miteinander verglichen. Dabei wird eine CS-Atmung registriert, wenn die Periodenlängen zwischen benachbarten Ereignissen um weniger als 40% und vorzugsweise weniger als 30% und besonders bevorzugt weniger als 20% voneinander abweichen. Dabei können zwischen den benachbarten Ereignissen auch Ereignisse liegen, welche insbesondere für die Ermittlung und den vergleich der Periodenlängen nicht berücksichtigt werden.

In einer vorteilhaften Weiterbildung ist die Überwachungseinrichtung dazu geeignet und ausgebildet ist, wenigstens eine Ereignisanalyse zur Erkennung eines für eine Cheyne-Stokes-Atmung charakteristischen Auftretens von wenigstens drei zeitlich aufeinander folgenden Ereignissen durchzuführen und dazu wenigstens eine erste Periodenlänge zwischen einem ersten Ereignis und einem zweiten Ereignis und eine zweite Periodenlänge zwischen einem zweiten Ereignis und einem dritten Ereignis zu ermitteln und wenigstens teilweise miteinander zu vergleichen und das Vorliegen einer Cheyne-Stokes-Atmung zu registrieren, wenn die erste Periodenlänge und die zweite Periodenlänge um weniger als 40% voneinander abweichen.

Bevorzugt ist die Überwachungseinrichtung dazu geeignet und ausgebildet, wenigstens eine Ereignisanalyse von wenigstens vier zeitlich aufeinander folgenden Ereignissen durchzuführen. Die Ereignisanalyse dient dabei zur Erkennung eines Auftretens der vier Ereignisse, welches charakteristisch für eine CS-Atmung ist. Für die Ereignisanalyse ist die Überwachungseinrichtung dazu geeignet und ausgebildet, wenigstens eine erste Periodenlänge zwischen einem ersten Ereignis und einem zweiten Ereignis und eine zweite Periodenlänge zwischen einem zweiten Ereignis und einem dritten Ereignis und eine dritte Periodenlänge zwischen einem dritten Ereignis und einem vierten Ereignis zu ermitteln. Zudem ist die Überwachungseinrichtung dazu geeignet und ausgebildet, die ermittelten Periodenlängen wenigstens teilweise miteinander zu vergleichen. Die Überwachungseinrichtung ist zudem dazu geeignet und ausgebildet, das Vorliegen einer CS-Atmung zu registrieren, wenn der Vergleich der Periodenlängen ergibt, dass die erste Periodenlänge und die zweite Periodenlänge um weniger als 40% voneinander abweichen und das die zweite Periodenlänge und die dritte Periodenlänge um weniger als 40% voneinander abweichen.

Ein Vorteil ist dabei, dass eine Überwachungseinrichtung vorgesehen ist, mit der ein charakteristisches Auftreten von wenigstens vier zeitlich aufeinander folgenden Ereignissen zur Erkennung einer CS-Atmung herangezogen wird. Dadurch wird die Fehlerschwelle deutlich verbessert.

Besonders vorteilhaft für die Erkennung einer CS-Atmung ist auch, dass die Überwachungseinrichtung die vorgeschriebene Abweichung von maximal 40% jeweils für zwei benachbarte Periodenlängen separat kontrolliert. Durch die Bestimmung der relativen Abweichung der Periodenlängen voneinander ist eine erheblich zuverlässigere Erkennung einer CS-Atmung als beispielsweise eine Überwachung einer absoluten Abweichung der Periodenlängen möglich. Obwohl eine CS-Atmung in der Regel durch eine ähnliche Periodenlänge benachbarter Ereignisse geprägt ist, kann die Periodenlänge jedoch über mehrere Ereignisse hinweg stärker zu- oder abnehmen. Durch die Berücksichtigung einer relativen Abweichung von benachbarten Ereignissen wird diesem Zusammenhang Rechnung getragen und die Zuverlässigkeit der CS-Erkennung erhöht.

Möglich ist auch, dass die dritte Periodenlänge ohne das vierte Ereignis ermittelt wird. Das erfolgt z. B. durch eine Erkennung eines kombinierten Hyper-Hypoventilationsereignisses, wobei die dritte Periodenlänge dann der Ereignislänge entspricht. Möglich sind auch andere Verfahren, z. B. statistische Auswertungen von Ereignisfolgen. Die vierte Periodenlänge wird bevorzugt auch mit der dritten Periodenlänge verglichen, sodass anhand des Maßes der Abweichung voneinander eine CS-Atmung feststellbar ist.

Die Ereignisanalyse kann auch wenigstens ein fünftes oder ein sechstes Ereignis umfassen. Die Ereignisanalyse kann auch eine Vielzahl von Ereignissen umfassen. Möglich ist auch, dass ein großer Teil der Ereignisse oder auch alle Ereignisse während der Beatmung für die Ereignisanalyse herangezogen werden. Dabei kann der Vergleich der Periodenlängen benachbarter Ereignisse in der entsprechenden Sequenz fortgesetzt werden. Die Auswertung der Ereignisse kann aber auch nach den vier Ereignissen oder einer bestimmten Anzahl von Ereignissen einmalig oder mehrmalig oder fortlaufend wiederholt werden.

Im Sinne dieser Erfindung meint der Begriff Periodenlänge insbesondere die Zeit zwischen zwei vergleichbaren Zeitpunkten innerhalb zweier Ereignisse. Beispielsweise kann sich die Periodenlänge von einem Beginn oder einem Ende oder einer Mitte eines Ereignisses zum Beginn oder Ende oder der Mitte eines anderen Ereignisses erstrecken. Möglich ist auch, dass sich ein Ereignis anhand einer Funktion beschreiben lässt. Die Periodenlänge erstreckt sich dann vorzugsweise von einem definierten Wert der Funktion eines Ereignisses zu einem anderen definierten Wert der Funktion eines benachbarten Ereignisses. Beispielsweise erstreckt sich die Periodenlänge von einem Maximum eines Ereignisses zu einem Maximum des benachbarten Ereignisses.

In einer bevorzugten Ausgestaltung ist die Überwachungseinrichtung dazu geeignet und ausgebildet, das Vorliegen einer CS-Atmung zu registrieren, wenn die erste Periodenlänge und die zweite Periodenlänge sowie die zweite Periodenlänge und die dritte Periodenlänge jeweils um weniger als 35% voneinander abweichen. Besonders bevorzugt registriert die Überwachungseinrichtung eine CS-Atmung, wenn die erste Periodenlänge und die zweite Periodenlänge sowie die zweite Periodenlänge und die dritte Periodenlänge jeweils um weniger als 30% voneinander abweichen. Eine geringere Abweichung als Grenzwert ist möglich, z. B. 20% oder weniger.

Vorzugsweise erstrecken sich die Periodenlängen zwischen einer zeitlichen Mitte eines Ereignisses und einer zeitlichen Mitte eines nachfolgenden Ereignisses. Insbesondere ist die Überwachungseinrichtung dazu geeignet und ausgebildet, die Periodenlängen ausgehend von einer zeitlichen Mitte des Ereignisses zu berechnen. Dabei ist die Überwachungseinrichtung insbesondere dazu geeignet und ausgebildet, auch den Beginn und/oder das Ende eines Ereignisses zu bestimmen. Dazu werden insbesondere zeitliche Veränderungen des erfassten Beatmungsparameters herangezogen, welche charakteristisch für den Beginn und/oder die Mitte und/oder das Ende eines Ereignisses sind.

Es ist bevorzugt, das die Überwachungseinrichtung dazu geeignet und ausgebildet ist, die Ereignisse für die Ereignisanalyse in wenigstens zwei Relevanzklassen zu sortieren. Die zwei Relevanzklassen umfassen dabei insbesondere wenigstens eine erste Relevanzklasse mit Ereignissen, die für eine CS-Atmung relevant sind. Die erste Relevanzklasse kann auch Ereignisse umfassen, welche als Positiv-Merkmale und/oder Negativ-Merkmale für das Vorliegen einer CS-Atmung herangezogen werden können.

Insbesondere umfassen die zwei Relevanzklassen wenigstens eine zweite Relevanzklasse mit Ereignissen, welche für eine CS-Atmung irrelevant sind. Weitere Relevanzklassen mit einer anderen Gewichtung der Ereignisse können vorgesehen sein.

Eine solche Sortierung von Ereignissen hat den Vorteil, dass die weitere Ereignisanalyse effizienter und wesentlich unaufwendiger erfolgen kann, da beispielsweise irrelevante Ereignisse nicht weiter analysiert werden müssen.

Die Sortierung in die zweite Relevanzklasse kann auch indirekt im Sinne einer Nichtzuordnung zu der ersten Relevanzklasse erfolgen. Vorzugsweise ist die Überwachungseinrichtung dazu geeignet und ausgebildet, die Sortierung in die Relevanzklassen vor der Bestimmung der Periodenlänge durchzuführen. Insbesondere werden nur die Ereignisse der ersten Relevanzklasse der Ereignisanalyse unterzogen, soweit das Sortieren in die Relevanzklassen nicht zur Ereignisanalyse gezählt wird.

Vorzugsweise ist die Überwachungseinrichtung dazu geeignet und ausgebildet, eine zuvor erfolgte Typisierung der Ereignisse bei der Sortierung in die Relevanzklassen zu berücksichtigen. So kann beispielsweise je nach Ereignistyp eine Sortierung erfolgen.

Insbesondere umfasst die erste Relevanzklasse wenigstens die Ereignisse einer Hyperventilation und/oder einer Hypoventilation und/oder einer Hypopnoe und/oder einer Apnoe.

Die zweite Relevanzklasse umfasst insbesondere wenigstens die Ereignisse Einatmen, Ausatmen, Schnarchen, Schlucken, Sprechen, Schnäuzen und/oder Husten und/oder eine Beatmungsunterbrechung und/oder eine Leckage im Beatmungssystem.

In einer vorteilhaften Weiterbildung ist die Überwachungseinrichtung dazu geeignet und ausgebildet, für die Ereignisanalyse die Ereignisse der ersten Relevanzklasse in wenigstens zwei Validitätsklassen zu sortieren. Die zwei Validitätsklassen umfassen vorzugsweise eine erste Validitätsklasse und eine zweite Validitätsklasse. Die erste Validitätsklasse umfasst insbesondere Ereignisse, welche bei einer CS-Atmung üblicherweise auftreten. Die zweite Validitätsklasse umfasst insbesondere Ereignisse, welche üblicherweise bei einer CS-Atmung nicht auftreten.

Die erste Validitätsklasse umfasst insbesondere Ereignisse, welche als Positiv-Merkmale für das Vorliegen einer CS-Atmung herangezogen werden können. Die Ereignisse der zweiten Validitätsklasse können Ereignisse sein, welche indizieren, dass eine CS-Atmung gerade nicht vorliegt. Eine solche Sortierung der Ereignisse in wenigstens zwei Validitätsklassen vereinfacht die Abwägung, ob eine CS-Atmung vorliegt oder nicht.

Vorzugsweise umfasst die erste Validitätsklasse wenigstens die Ereignisse einer zentralen Apnoe und/oder einer zentralen Hypopnoe.

Die zweite Validitätsklasse umfasst vorzugsweise wenigstens die Ereignisse einer obstruktiven Apnoe und/oder einer obstruktiven Hypopnoe.

Besonders bevorzugt ist die Überwachungseinrichtung dazu geeignet und ausgebildet, das Vorliegen einer CS-Atmung zu registrieren, wenn wenigstens drei analysierte Ereignisse und insbesondere drei der wenigstens vier analysierten Ereignisse zeitlich aufeinander folgen und wenigstens zwei Bedingungen erfüllen. Die erste Bedingung ist, dass wenigstens zwei der der wenigstens drei Ereignisse der ersten Validitätsklasse entsprechen. Die zweite Bedingung ist, dass maximal ein Ereignis der wenigstens drei Ereignisse der zweiten Validitätsklasse entspricht. Weitere Bedingungen sind möglich, welche insbesondere das Auftreten bestimmter Typen von Ereignissen regeln. Eine solche Ausgestaltung hat den Vorteil, dass die Überwachungseinrichtung das Vorliegen einer CS-Atmung mittels einer Abwägung von Positiv- und Negativ- Merkmalen zuverlässig registrieren kann. Insbesondere folgen die drei Ereignisse zeitlich direkt aufeinander. Dabei kann zwischen den direkt folgenden Ereignissen wenigstens ein Ereignis liegen, welches für die Ereignisanalyse nicht berücksichtigt wird.

In einer weiteren bevorzugten Ausgestaltung ist die Überwachungseinrichtung dazu geeignet und ausgebildet, das Vorliegen einer CS-Atmung zu registrieren, wenn eine erste und eine zweite und eine dritte Periodenlänge jeweils wenigstens 35 Sekunden und vorzugsweise wenigstens 40 Sekunden andauern. Vorzugsweise dauern die erste und die zweite und die dritte Periodenlänge jeweils wenigstens 35 Sekunden und vorzugsweise wenigstens 40 Sekunden an. Besonders bevorzugt ist die Überwachungseinrichtung auch dazu geeignet und ausgebildet, das Vorliegen einer CS-Atmung zu registrieren, wenn die erste und die zweite und dritte Periodenlänge jeweils maximal 95 Sekunden und vorzugsweise maximal 90 Sekunden andauern. Da eine CS-Atmung in der Regel durch in bestimmten Zeitabständen auftretende Ereignisse charakterisiert ist, ist eine Eingrenzung der Periodenlänge besonders vorteilhaft für die Erkennung der CS-Atmung. Es kann auch wenigstens eine der Periodenlängen durch ein statistisches Verfahren oder durch Heranziehung wenigstens einer Ereignislänge bestimmt werden.

Es ist möglich und bevorzugt, dass die Überwachungseinrichtung dazu geeignet und ausgebildet ist, das Vorliegen einer CS-Atmung zu registrieren, wenn eine zeitliche Dauer jeweils eines der für die Ereignisanalyse berücksichtigten Ereignisse maximal 80% der Periodenlänge beträgt. Insbesondere wenn eine zeitliche Dauer jeweils eines der wenigstens 4 Ereignisse maximal 80% der Periodenlänge beträgt. Insbesondere wird die CS-Atmung registriert, wenn die zeitliche Dauer der einzelnen Ereignisse jeweils maximal 70% der Periodenlänge beträgt. Der Grenzwert kann auch geringer sein, z. B. 60% oder 50% oder darunter. Durch eine solche Ausgestaltung werden die Dauern der einzelnen Ereignisse in eine Relation zu der ereignisfreien Zeit zwischen den Ereignissen gesetzt, welche sich gut zur Erkennung einer CS-Atmung eignet.

Es ist möglich, dass die Überwachungseinrichtung dazu geeignet und ausgebildet ist, ein Flowsignal und/oder ein Drucksignal zu überwachen und anhand wenigstens eines Algorithmus auszuwerten, um ein Ereignis zu typisieren. Das Flowsignal und/oder das Drucksignal werden dabei von wenigstens einer der Überwachungseinrichtung zugeordneten Sensoreinrichtung bereitgestellt. Die Sensoreinrichtung ist dabei zur Erfassung wenigstens einer Floweigenschaft und/oder wenigstens einer Druckeigenschaft des Luftstroms für die Beatmung vorgesehen. Die Auswertung von Flowsignalen bzw. Drucksignalen ermöglicht eine zuverlässige Erkennung und Typisierung von Ereignissen der Atmung.

Besonders bevorzugt ist die Überwachungseinrichtung auch dazu geeignet und ausgebildet, anhand des Flowsignals und/oder des Drucksignals wenigstens einen Beatmungsparameter zu registrieren, wie z.B. die Atemfrequenz, das Atemzugvolumen, das Atemminutenvolumen, den Inspirationsflow und/oder den Inspirationsdruck und/oder den Atemwegswiderstand.

Die Überwachungseinrichtung kann dazu geeignet und ausgebildet sein, bei einer registrierten CS-Atmung einen entsprechenden Hinweis an einen Benutzer auszugeben. Beispielsweise kann der Hinweis mittels einer Anzeigeeinrichtung optisch und/oder akustisch angezeigt werden. Der Hinweis kann beispielsweise auch mittels einer Schnittstelle an wenigstens eine externe Datenverarbeitungseinrichtung ausgegeben werden. Ein solcher Hinweis ist besonders vorteilhaft, da in Kenntnis der CS-Atmung die Beatmungsbehandlung entsprechend angepasst werden kann. Andererseits kann beim Ausbleiben des Hinweises ein entsprechender Erfolg der Beatmungsbehandlung festgestellt werden.

Die Überwachungseinrichtung ist alternativ auch dazu geeignet und ausgebildet, bei einer registrierten Cheyne-Stokes-Atmung die Beatmungseinrichtung auf wenigstens einen Beatmungsmodus zur Beatmung bei einer Cheyne-Stokes-Atmung einzustellen.

Die Überwachungseinrichtung kann auch dazu geeignet und ausgebildet sein, bei einer registrierten CS-Atmung einen entsprechenden Hinweis an einen Benutzer auszugeben und/oder die CS-Atmung in einer fortlaufenden Auszeichnung der Atmung zu dokumentieren. Beispielsweise kann der Hinweis mittels einer Anzeigeeinrichtung optisch und/oder akustisch angezeigt werden. Der Hinweis kann beispielsweise auch mittels einer Schnittstelle an wenigstens eine externe Datenverarbeitungseinrichtung ausgegeben werden. Ein solcher Hinweis und/oder eine Speicherung der registrierten CS-Atmung ist besonders vorteilhaft, da in Kenntnis der CS-Atmung die Beatmungsbehandlung entsprechend angepasst werden kann. Beispielsweise wird bei registrierter CS-Atmung eine Erhöhung des IPAP und/oder eine Erhöhung des EPAP unterdrückt. Alternativ wird eine Empfehlung ausgegeben den IPAP und/oder den EPAP abzusenken.

Die Überwachungseinrichtung ist alternativ auch dazu geeignet und ausgebildet, bei einer registrierten Cheyne-Stokes-Atmung die Beatmungseinrichtung auf wenigstens einen Beatmungsmodus zur Beatmung bei einer Cheyne-Stokes-Atmung einzustellen, bei dem zumindest ein Druck (IPAP oder EPAP) vermindert wird.

Die Überwachungseinrichtung kann auch dazu geeignet und ausgebildet sein, bei einer registrierten CS-Atmung diese als eine entsprechende Atemstörung in einer Beatmungsstatistik zu berücksichtigen. Eine solche statistische Auswertung des Auftretens einer CS-Atmung ist hilfreich, um beispielsweise Grunderkrankungen zu erkennen.

Es kann vorgesehen sein, das die Überwachungseinrichtung dazu geeignet und ausgebildet ist, bei einer registrierten CS-Atmung die Beatmungseinrichtung auf wenigstens einen für die Beatmung bei einer CS-Atmung optimierten Beatmungsmodus einzustellen. Eine solche Ausgestaltung hat den Vorteil, dass die Beatmungseinrichtung automatisch angepasst wird, wenn eine CS-Atmung erkannt wird. Dadurch erhält der Benutzer sofort eine für ihn optimierte Beatmung. Insbesondere umfasst der Beatmungsmodus bei einer CS-Atmung wenigstens eine Anpassung eines Beatmungsparameters und vorzugsweise eine antizyklische Anpassung des Druckhubs. Die automatische Einstellung des Beatmungsmodus kann manuell deaktivierbar sein kann. Vorzugsweise wird die Einstellung des Beatmungsmodus in der Beatmungsstatistik vermerkt, sodass eine Kontrolle erfolgen kann, ob die Umstellung vorteilhaft für die Behandlung ist.

Das erfindungsgemäße Verfahren dient zum Betreiben eines Beatmungsgerätes mit wenigstens einer Beatmungseinrichtung zur Erzeugung eines Luftstroms für die Beatmung. Das Beatmungsgerät umfasst wenigstens eine Überwachungseinrichtung zur Erfassung wenigstens eines Beatmungsparameters und zur Typisierung von Ereignissen in der Atmung anhand einer Überwachung des Beatmungsparameters. Dabei wird anhand wenigstens einer Ereignisanalyse wenigstens ein Auftreten zeitlich aufeinander folgender Ereignisse erkannt, welches charakteristisch für eine Cheyne-Stokes-Atmung ist. Für die Ereignisanalyse werden Periodenlängen zwischen den zeitlich aufeinander folgenden Ereignissen ermittelt. Die Periodenlängen werden wenigstens teilweise miteinander verglichen. Das Vorliegen einer Cheyne-Stokes-Atmung wird dann registriert, wenn die verglichenen Periodenlängen um weniger als 40% voneinander abweichen.

Erfindungsgemäß ist die Überwachungseinrichtung dazu geeignet und ausgebildet, wenigstens eine Ereignisanälyse zur Erkennung eines für eine Cheyne-Stokes-Atmung charakteristischen Auftretens zeitlich aufeinander folgender Ereignisse durchzuführen und dazu Atmungsamplituden für zumindest drei zeitlich aufeinander folgenden Atemereignisse (Einatmung und Ausatmung) zu ermitteln und die ermittelten Atmungsamplituden wenigstens teilweise miteinander zu vergleichen und das Vorliegen einer Cheyne-Stokes-Atmung zu registrieren, wenn die verglichenen Atmungsamplitude um zumindest 25% voneinander abweichen. Hierbei wird bevorzugt die Amplitude aus dem Fluss oder Volumen von Atemgas bestimmt und ergibt sich aus dem Wechsel von einströmendem und ausströmendem Atemgas. Beispielhaft wird die erste Amplitude mit der dritten Amplitude verglichen und eine CS-Atmung erkannt, wenn die die erste Amplitude um zumindest 25% von der dritten Amplitude abweicht. Beispielhaft wird auch die erste Amplitude mit der dritten Amplitude verglichen und eine CS-Atmung erkannt, wenn die die erste Amplitude um zumindest 50% von der dritten Amplitude abweicht. Eine CS-Atmung wird ebenfalls erkannt, wenn die die erste Amplitude um zumindest 75% von der dritten Amplitude abweicht. Beispielsweise können zwischen den zumindest drei zeitlich aufeinander folgenden Atemereignissen (Einatmung und Ausatmung) oder innerhalb der drei Atemereignisse auch Phasen von zumindest zeitweiser sehr geringer Amplitude oder ausbleibender Amplitude zeigen. Eine CS-Atmung wird auch dann erkannt, wenn zwischen den zumindest drei zeitlich aufeinander folgenden Atemereignissen (Einatmung und Ausatmung) oder innerhalb der drei Atemereignisse Phasen von zumindest zeitweiser sehr geringer Amplitude oder ausbleibender Amplitude von zumindest 8 Sekunden auftreten. Erfindungsgemäß erfolgt ein Ermitteln, Speichern und Anzeigen einer Amplitudenstärke der CS-Atmung, also bestehend aus Hypopnoen versus Apnoen.

Erfindungsgemäß erfolgt eine Ermittlung eines Trends der Amplitudenstärke über Stunden oder Tage oder Wochen als Indikator für Veränderung einer Erkrankung, z. B. Herzinsuffizienz oder neurologische Erkrankung.

Erfindungsgemäß wird die erkannte CS-Atmung durch Analyse von Pulsoximeter-Signalen bestätigt oder verworfen. Als Pulsoximeter-Signale dienen zyklische Schwankung von SpO2 oder der Pulsfrequenz oder der Pulswellenamplitude oder der Pulstransitzeit (PTT). Das Pulsoximeter wird dabei über eine Schnittstelle mit dem Beatmungsgerät verbunden, um die Daten zeitkorreliert aufzeichnen zu können.

Erfindungsgemäß erfolgt ein Speichern und Anzeigen der ermittelten Periodendauer.

Erfindungsgemäß erfolgt die Ermittlung eines Trends der Periodendauer über Stunden oder Tage oder Wochen, da die Periodendauer ein Indikator für Veränderung einer Erkrankung, z. B. Herzinsuffizienz oder neurologische Erkrankung ist.

Das Verfahren bietet eine unaufwendige Möglichkeit, während der Beatmung durch ein Beatmungsgerät eine CS-Atmung zuverlässig zu erkennen. Besonders bevorzugt ist das Verfahren so ausgebildet, dass es zum Betreiben des erfindungsgemäßen Beatmungsgerätes sowie dessen Weiterbildungen einsetzbar ist. Dadurch kann das Verfahren besonders gut umgesetzt und dessen Vorteile genutzt werden.

Weitere Vorteile und Merkmale der vorliegenden Erfindung ergeben sich aus der Beschreibung des Ausführungsbeispiels, welches mit Bezug auf die beiliegenden Figuren im Folgenden erläutert wird.

In den Figuren zeigen:
Fig. 1 ein erfindungsgemäßes Beatmungsgerät; und
Fig. 2 eine skizzierte Ereignisanalyse.

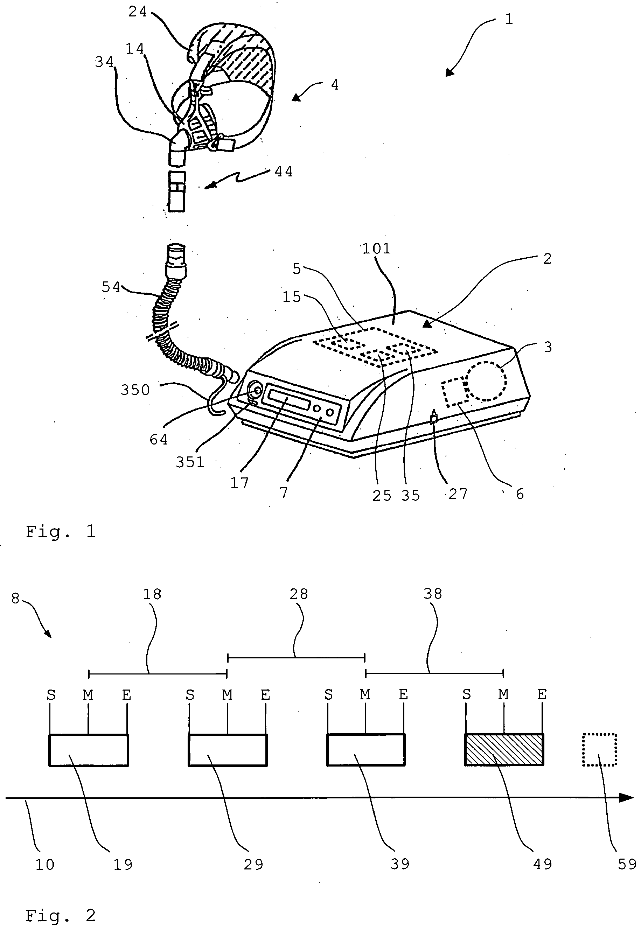

Die Figur 1 zeigt ein erfindungsgemäßes Beatmungsgerät 1 in einer perspektivischen Darstellung. Das Beatmungsgerät 1 ist insbesondere ein Heimbeatmungsgerät. Das Beatmungsgerät 1 ist zum Betrieb nach dem erfindungsgemäßen Verfahren vorgesehen und ausgebildet.

Das Beatmungsgerät 1 umfasst eine von einem Gehäuse 101 umgegebene Beatmungseinrichtung 2 mit einer Gebläseeinrichtung 3 zur Erzeugung eines Luftstromes für die Beatmung. Zur Steuerung der Beatmungseinrichtung 2 ist eine Überwachungseinrichtung 5 mit einer Speichereinrichtung 15, einem Controller 25 und einer Sensoreinrichtung 35 vorgesehen.

Die Bedienung und Einstellung des Beatmungsgerätes 1 erfolgt über eine Bedieneinrichtung 7 mit einer Anzeigeeinrichtung 17 und einer Schnittstelle für den Datenaustausch 27. Zur Erhöhung des Feuchtegehaltes der Atemluft ist eine Befeuchte-. Einrichtung 6 vorgesehen.

Das Beatmungsgerät 1 weist eine Atemschnittstelle 4 auf, um den Luftstrom einem Benutzer zur Beatmung zuzuführen. Die Atemschnittstelle ist vorzugsweise als ein bei Beatmungsgeräten übliches Patienteninterface ausgebildet und beispielsweise als eine Vollgesichtsmaske oder als ein Nasal-Pillow oder als ein Tubus oder als eine Larynxmaske ausgestaltet sein. Die hier gezeigte Atemschnittstelle 4 ist eine als Nasalmaske ausgebildete Atemmaske 14. Zur Fixierung der Atemmaske 14 ist eine Kopfhaube 24 vorgesehen.

Zur Verbindung der Atemschnittstelle 4 mit der Beatmungseinrichtung 2 ist ein Verbindungsschlauch 54 vorgesehen, der mittels einer Koppeleinrichtung 64 mit der Beatmungseinrichtung 2 verbunden wird. Über ein Kuppelungselement 34 wird der Verbindungsschlauch 54 mit der Atemschnittstelle 4 verbunden. Zwischen dem Verbindungschlauch 54 und dem Kuppelungselement 12 ist ein Ausatmungselement 44 angeordnet, welches ein Ventil umfasst oder als ein solches ausgebildet ist. Das Ausatmungselement 44 ist insbesondere dazu vorgesehen, während der Ausatmung des Benutzers Rückatmen in das Beatmungsgerät 1 zu verhindern.

Die Überwachungseinrichtung 5 mit der Sensoreinrichtung 35 wirkverbunden, welche einen oder mehrere Sensoren zur Erfassung von Größen, welche charakteristisch für den Beatmungsparameter sind, aufweist. Beispielhaft hat die Sensoreinrichtung 35 einen hier nicht gezeigten Drucksensor, welcher die Druckverhältnisse im Bereich der Atemschnittstelle erfasst. Dazu ist der Drucksensor über einen Druckmessschlauch 350 mit der Atemschnittstelle 4 strömungsverbunden.

Der Druckmessschlauch 350 erstreckt sich dabei von der Atemschnittstelle 4 entlang der Verbindungsschlauches 54 zu einem Eingangsstutzen 351 am Gehäuse 101. Über den Eingangsstutzen 351 ist der Druckmessschlauch 350 mit dem Drucksensor der Sensoreinrichtung 35 strömungsverbunden.

Die Flowmessung erfolgt dabei über eine Drossel, welche im Hauptkanal der Strömungsverbindung zwischen Gebläseeinrichtung 3 und Atemschnittstelle 4 angeordnet ist. Möglich ist auch eine Anordnung in einem Nebenkanal. Um weitere Beatmungsparameter überwachen zu können, kann die Sensoreinrichtung 35 auch mit Sensoren zur Messung der Atemexkursion, zur Messung einer Sauerstoffsättigung des Blutes und/oder zur Messung einer EEG-, einer EMG-, einer EOG- oder einer EKG-Aktivität ausgestattet sein.

Des Weiteren weist die Überwachungseinrichtung 5 einen Controller 25 zur Ansteuerung der Gebläseeinrichtung 3 auf. Der Controller 25 stellt einen notwendigen Minimaldruck bereit und kompensiert Druckschwankungen, die durch die Atemtätigkeit des Benutzers bedingt sind. Beispielsweise erfasst der Controller 25 auch den gegenwärtigen Druck in der Atemmaske 14 und regelt die Leistung der Gebläseeinrichtung 3 entsprechend nach, bis ein gewünschter Beatmungsdruck anliegt.

Das hier gezeigte Beatmungsgerät 1 kann als ein Fix-Level-Gerät oder auch als ein Automatic-Level-Gerät ausgebildet sein. Insbesondere erfolgt dabei durch die Überwachungseinrichtung 5 eine Regelung auf Sollwerte, welche zuvor anhand der charakteristischen Atmung eines Benutzers individuell berechnet und festgelegt worden sind.

Es ist auch möglich, das die Beatmungseinrichtung 2 dynamisch und insbesondere je nach Atemphase des Benutzers angepasst wird. Beispielsweise kann anhand der Überwachungseinrichtung 5 ein Atemphasenwechsel erkannt werden, sodass je nach Atemphase ein höherer bzw. niedrigerer Druck bereitgestellt werden kann. Beispielsweise kann das Beatmungsgerät 1 als ein CPAP- oder APAP-Gerät ausgebildet sein. Das Beatmungsgerät 1 kann auch als ein Bilevel-Gerät ausgebildet sein. Beispielsweise reagiert das Beatmungsgerät 1 auf bestimmte Atemereignisse, wie z.B. Schnarchen, Atemabflachungen und/oder obstruktive Druckspitzen mit entsprechenden Einstellungen der Beatmungsparameter.

Mit dem hier gezeigten Beatmungsgerät 1 werden Ereignisse, die in der Atmung bzw. während der Beatmung auftreten, erkannt und typisiert. Dazu erfasst die Sensoreinrichtung 35 einen oder mehrere Beatmungsparameter und liefert entsprechende Signale an die Überwachungseinrichtung 5, wie z.B. ein Flowsignal und/oder ein Drucksignal. Die Überwachungseinrichtung 5 analysiert die Signale mittels geeigneter Algorithmen, sodass charakteristische Signalverläufe erkannt und als ein Ereignis typisiert werden können. Dabei kann beispielsweise auf eine Parameterextraktion in Bezug auf Pegel und Amplitudenwerte, Zeitabstände, Einhüllende, Nulldurchgänge und Steigungen zurückgegriffen werden. Bei einer Analyse im Hinblick auf Zeitmerkmale wird beispielsweise bei einer Parametextraktion auf Periodizitäten und Frequenzen zurückgegriffen.

Die erkannten Ereignisse können dann in der Speichereinrichtung 15 abgelegt und für eine Beatmungsstatistik herangezogen werden. Ein Vorteil der Ereigniserkennung ist, dass auf Basis der typisierten Ereignisse eine Anpassung der Beatmung erfolgen kann, z.B. eine automatische Druckanhebung bei einer obstruktiven Apnoe. Zudem kann in einem gewissen Maße eine Diagnostik bestimmter Atemstörungen erfolgen.

Ein besonderer Vorteil des hier gezeigten Beatmungsgerätes 1 ist die Überwachungseinrichtung 5, welche die erkannten Ereignisse weiter analysiert und anhand eines charakteristischen Auftretens der Ereignisse eine CS-Atmung registriert. Eine solche Ereignisanalyse 8 ist beispielhaft in der Figur 2 skizziert.

Die Figur 2 zeigt fünf beispielhafte Ereignisse 19, 29, 39, 49, 59, die während einer Zeit 10 erfasst und typisiert wurden. Das erste und das zweite sowie das dritte Ereignis 19, 29 ,39 entsprechen hier jeweils einer in der Atmung aufgetretenen zentralen Apnoe. Das vierte Ereignis 49 entspricht einer obstruktiven Apnoe. Das fünfte Ereignis 59 steht hier für ein während der Beatmung aufgetretenes Schnarchen. Zwischen den einzelnen Ereignissen 19, 29, 39, 49, 59 können weitere, hier nicht gezeigte Ereignisse aufgetreten und erfasst worden sein, wie z.B. eine Hyperventilation.

Für die Ereignisanalyse 8 sortiert die Überwachungseinrichtung 5 die Ereignisse zunächst nach ihrer Relevanz bezüglich einer CS-Atmung. Relevant sind dabei beispielsweise eine zentrale Hypopnoe sowie eine zentrale Apnoe. Die Überwachungseinrichtung 5 stuft daher das erste, zweite, dritte und vierte Ereignis als relevant für eine CS-Erkennung ein und sortiert diese z.B. in eine erste Relevanzklasse. Das als Schnarchen typisierte fünfte Ereignis 59 wird als nicht relevant für die CS-Erkennung angesehen und von der Überwachungseinrichtung 5 z.B. in eine zweite Relevanzklasse einsortiert. Für die weitere Ereignisanalyse 8 berücksichtigt die Überwachungseinrichtung 5 dann nur noch die Ereignisse der ersten Relevanzklasse.

Anschließend analysiert die Überwachungseinrichtung 5 das Auftreten der relevanten Ereignisse über die Zeit 10. Dazu ermittelt die Überwachungseinrichtung 5 den Zeitpunkt des Starts S, den Zeitpunkt des Endes E sowie die zeitliche Mitte jedes der relevanten Ereignisse. Anschließend werden die Periodenlängen bestimmt, welche sich jeweils von der Mitte M eines Ereignisses zur zeitlichen Mitte M des nachfolgenden Ereignisses erstrecken. So ergibt sich eine erste Periodenlänge 18 zwischen dem ersten und zweiten Ereignis 19, 29, eine zweite Periodenlänge 28 zwischen dem zweiten und dritten Ereignis 29, 39 sowie eine dritte Periodenlänge 38 zwischen dem dritten und vierten Ereignis 39, 49.

In einem nächsten Schritt vergleicht die Überwachungseinrichtung 5 die erste Periodenlänge 18 mit der zweiten Periodenlänge 28. Ergibt der Vergleich eine maximale Abweichung von 30% der Periodenlängen 18, 28 voneinander, wird dieses als ein Indiz für ein CS-Atmung registriert. Anschließend wird auch die Abweichung zwischen der zweiten Periodenlänge 28 und der dritten Periodenlänge 38 ermittelt und bei einer maximalen Abweichung von 30% als weiteres Indiz für das Vorliegen einer CS-Atmung gewertet. Dabei können für die Ereignisanalyse 8 auch noch mehr als vier Ereignisse berücksichtigt analysiert werden, sodass sich auch der Vergleich über weitere benachbarte Periodenlängen erstreckt.

Als weiteres Indiz für das Vorliegen einer CS-Atmung zieht die Überwachungseinrichtung 5 den jeweiligen Wert der Periodenlänge zwischen den Ereignissen heran. Dabei wird vom Vorliegen einer CS-Atmung ausgegangen, wenn die Periodenlängen jeweils wenigstens 40 Sekunden und maximal 90 Sekunden betragen.

Nachfolgend prüft die Überwachungseinrichtung 5 das Verhältnis zwischen der zeitlichen Dauer eines Ereignisses und der zugehörigen Periodenlänge. Beispielsweise wird die Dauer des ersten Ereignisses 19 vom Start S bis zum Ende E ermittelt und mit der ersten Periodenlänge 18 verglichen. Die Überwachungseinrichtung 5 registriert dabei ein Indiz für eine CS-Atmung, wenn die zeitliche Dauer maximal 70% der dazugehörigen Periodenlänge beträgt. Entsprechend wird die zeitliche Dauer des zweiten und dritten Ereignisses 29, 39 mit der jeweils zugehörigen Periodenlänge 28, 38 verglichen und analysiert.

Anschließend werden die als relevant einsortierten Ereignisse von der Überwachungseinrichtung 5 validiert. Dabei werden Ereignisse als valide eingestuft, wenn sie bei einer CS-Atmung üblicherweise vorkommen. Als invalide werden die Ereignisse eingestuft, die üblicherweise nicht bei einer CS-Atmung auftreten oder bei deren einmaligen oder mehrmaligen Auftreten eine CS-Atmung zu verneinen ist. Als valide Ereignisse gelten beispielsweise eine zentrale Apnoe oder Hypopnoe. Als Invalide gilt beispielsweise eine obstruktive Apnoe oder Hypopnoe.

Dabei sortiert die Überwachungseinrichtung 5 die Ereignisse in eine erste Validitätsklasse und eine zweite Validitätsklasse. Die erste Validitätsklasse umfasst hier das erste, zweite und dritte Ereignis 19, 29, 39, welche jeweils als Apnoe typisiert wurden. Das als obstruktive Hypopnoe typisierte vierte Ereignis 49 wird in die zweite Validitätsklasse einsortiert.

Anschließend berücksichtigt die Überwachungseinrichtung 5 die Anzahl der in die erste und in die zweite Validitätsklasse einsortierten Ereignisse zusammen mit der Gesamtzahl aller in der Ereignisanalyse berücksichtigten Ereignisse im Sinne einer Schlüssigkeitsprüfung. In dem hier gezeigten Beispiel mit vier relevanten Ereignissen wird das Vorliegen einer CS-Atmung angenommen, wenn von drei Ereignissen wenigstens zwei als valide Ereignisse und maximal eines als invalides Ereignis eingestuft werden.

Die Unterscheidung zwischen einer als valides Ereignis einstufbaren zentralen Apnoe und einer invaliden obstruktiven Apnoe vollzieht die Überwachungseinrichtung 5 vorzugsweise anhand einer Analyse des Flowsignals und des Drucksignals. Die Signale werden zwischen der Atemschnittstelle 4 und dem Ausatmungselement 44 abgegriffen. Der Beginn einer Apnoe wird anhand einer Flowschwankung und das Ende der Apnoe anhand einer Druckschwankung erkannt.

Das Ende der Apnoe kann aber auch durch das Auftreten einer Druck- oder Flowschwankung erkannt werden. Die Abgrenzung einer obstruktiven Apnoe erfolgt dabei durch das Auftreten eines kurzen, zusätzlichen Druckanstieges am Ende der Apnoe. Um den Druckanstieg von möglichen Artefakten, wie z.B. Bewegungen oder Herzschlag des Benutzers, abzugrenzen, kann ein entsprechender Grenzwert festgelegt sein. Zudem ist der Druckanstieg so kurz, dass er erheblich kürzer als typische Atemperioden ist und somit von diesen abgegrenzt werden kann.

Die Ereignisanalyse 8 erfolgt vorzugsweise unter der Berücksichtigung von Übereinkünften zur Erkennung von Atemstörungen. Beispielsweise beschreibt das AASM-Manual entsprechende Regeln zur Ereigniserkennung und Klassifikation, die bei der Auswertung zu berücksichtigen sind. Die Überwachungseinrichtung 5 ist insbesondere dazu geeignet und ausgebildet, diese Regeln bei der Ereignisanalyse zur Erkennung der CS-Atmung in entsprechender Weise zu berücksichtigen. Es sind entsprechende Abfragen und/oder Algorithmen in der Überwachungseinrichtung 5 hinterlegt.

Beispielsweise müssen zur Erkennung einer CS-Atmung mindestens fünf zentrale Apnoen und/oder zentrale Hypopnoen pro Stunde während des Schlafes des Benutzers in Verbindung mit dem typischen Muster eines Ansteigens und Abflachens der Atmung über mindestens zwei Stunden Beobachtungszeitraum auftreten. Zudem ist es möglich, dass zentrale Apnoen, welche während einer bereits erkannten CS-Atmung auftreten, als individuelle Apnoen berücksichtigt werden und entsprechend in die Ereignisanalyse 8 einfließen.

Ergibt die Ereignisanalyse 8 das Vorliegen einer CS-Atmung, kann diese anhand eines entsprechenden Eintrags in einer Beatmungsstatistik hinterlegt werden. Der Benutzer kann die Beatmungsstatistik z.B. über die Schnittstelle 27 abrufen und zur Beurteilung der Beatmungstherapie heranziehen. Je nach Häufigkeit der CS-Atmung kann dann auch eine manuelle oder programmunterstützte Umstellung der jeweiligen Beatmungsparameter erfolgen. Die Überwachungseinrichtung 5 kann bei einem Auftreten der CS-Atmung auch eine automatische Adaption der Beatmungseinrichtung 2 vornehmen und entsprechende Sollwerte für Beatmungsparameter neu berechnen. Beispielsweise kann ein bestimmter Therapiemodus eingestellt werden, der die CS-Atmung durch antizyklische Regelung des Druckhubs vorsieht, einstellen.

### Bezugszeichenliste:

- 1: Beatmungsgerät
- 2: Beatmungseinrichtung
- 3: Gebläseeinrichtung
- 4: Atemschnittstelle
- 5: Überwachungseinrichtung
- 6: Befeuchte-Einrichtung
- 7: Bedieneinrichtung
- 8: Ereignisanalyse
- 10: Zeit
- 14: Atemmaske
- 15: Speichereinrichtung
- 17: Anzeigeeinrichtung
- 18: Periodenlänge
- 19: Ereignis
- 24: Kopfhaube
- 25: Controller
- 27: Schnittstelle
- 28: Periodenlänge
- 29: Ereignis
- 34: Kupplungselement
- 35: Sensoreinrichtung
- 38: Periodenlänge
- 39: Ereignis
- 44: Ausatmungselement
- 49: Ereignis
- 54: Verbindungsschlauch
- 59: Ereignis
- 64: Koppeleinrichtung
- 101: Gehäuse
- 350: Druckmessschlauch
- 351: Eingangsstutzen

## Patentansprüche

1. Beatmungsgerät (1) mit wenigstens einer Beatmungseinrichtung (2) zur Erzeugung eines Luftstroms für die Beatmung und mit wenigstens einer Überwachungseinrichtung (5) zur Erfassung wenigstens eines Beatmungsparameters und zur Typisierung von Ereignissen (19, 29, 39, 49, 59) in der Atmung anhand einer Überwachung des Beatmungsparameters, wobei die Überwachungseinrichtung (5) dazu geeignet und ausgebildet ist, wenigstens eine Ereignisanalyse (8) zur Erkennung eines für eine Cheyne-Stokes-Atmung charakteristischen Auftretens zeitlich aufeinander folgender Ereignisse durchzuführen und dazu Periodenlängen (18) zwischen den zeitlich aufeinander folgenden Ereignissen (19, 29, 39, 49, 59) zu ermitteln und die ermittelten Periodenlängen (18) miteinander zu vergleichen und **dadurch gekennzeichnet, dass** die ermittelten Periodenlängen (18) als das Vorliegen einer Cheyne-Stokes-Atmung registriert werden, wenn die verglichenen Periodenlängen (18) um weniger als 40% voneinander abweichen.

2. Beatmungsgerät (1) nach Anspruch 1, wobei die Überwachungseinrichtung (5) dazu geeignet und ausgebildet ist, wenigstens eine Ereignisanalyse (8) zur Erkennung eines für eine Cheyne-Stokes-Atmung charakteristischen Auftretens von wenigstens drei zeitlich aufeinander folgenden Ereignissen (19, 29, 39, 49, 59) durchzuführen und dazu wenigstens eine erste Periodenlänge (18) zwischen einem ersten Ereignis und einem zweiten Ereignis und eine zweite Periodenlänge (18) zwischen einem zweiten Ereignis und einem dritten Ereignis zu ermitteln und miteinander zu vergleichen und das Vorliegen einer Cheyne-Stokes-Atmung zu registrieren, wenn die erste Periodenlänge (18) und die zweite Periodenlänge (18) um weniger als 40% voneinander abweichen.

3. Beatmungsgerät (1) nach einem der vorhergehenden Ansprüche, wobei die Überwachungseinrichtung (5) dazu geeignet und ausgebildet ist, wenigstens eine Ereignisanalyse (8) zur Erkennung eines für eine Cheyne-Stokes-Atmung charakteristischen Auftretens von wenigstens vier zeitlich aufeinander folgenden Ereignissen (19, 29, 39, 49, 59) durchzuführen und dazu wenigstens eine erste Periodenlänge (18) zwischen einem ersten Ereignis und einem zweiten Ereignis und eine zweite Periodenlänge (18) zwischen einem zweiten Ereignis und einem dritten Ereignis und eine dritte Periodenlänge (18) zwischen einem dritten Ereignis und einem vierten Ereignis zu ermitteln und miteinander zu vergleichen und das Vorliegen einer Cheyne-Stokes-Atmung zu registrieren, wenn die erste Periodenlänge (18) und die zweite Periodenlänge (18) sowie die zweite Periodenlänge (18) und die dritte Periodenlänge (18) jeweils um weniger als 40% voneinander abweichen.

4. Beatmungsgerät (1) nach dem vorhergehenden Anspruch, wobei die Überwachungseinrichtung (5) dazu geeignet und ausgebildet ist, das Vorliegen einer Cheyne-Stokes-Atmung zu registrieren, wenn die erste Periodenlänge (18) und die zweite Periodenlänge (18) sowie die zweite Periodenlänge (18) und die dritte Periodenlänge (18) jeweils um weniger als 35% und insbesondere um weniger als 30% voneinander abweichen.

5. Beatmungsgerät (1) nach einem der vorhergehenden Ansprüche, wobei sich die Periodenlängen (18) zwischen einer zeitlichen Mitte eines Ereignisses und einer zeitlichen Mitte eines nachfolgenden Ereignisses erstrecken.

6. Beatmungsgerät (1) nach einem der vorhergehenden Ansprüche, wobei die Überwachungseinrichtung (5) dazu geeignet und ausgebildet ist, die Ereignisse in wenigstens zwei Relevanzklassen zu sortieren, umfassend wenigstens eine erste Relevanzklasse mit für eine Cheyne-Stokes-Atmung relevanten Ereignissen (19, 29, 39, 49, 59) und wenigstens eine zweite Relevanzklasse mit für eine Cheyne-Stokes-Atmung irrelevanten Ereignissen.

7. Beatmungsgerät (1) nach dem vorhergehenden Anspruch, wobei die erste Relevanzklasse wenigstens die Ereignisse (19, 29, 39, 49, 59) einer Hyperventilation und/oder einer Hypoventilation und/oder einer Hypopnoe und/oder einer Apnoe umfasst und /oder die zweite Relevanzklasse wenigstens die Ereignisse Einatmen, Ausatmen, Schnarchen, Schlucken, Sprechen, Schnäuzen und/oder Husten und/oder eine Beatmungsunterbrechung und/oder eine Leckage umfasst.

8. Beatmungsgerät (1) nach Anspruch 6 oder 7, wobei die Überwachungseinrichtung (5) dazu geeignet und ausgebildet ist, für die Ereignisanalyse (8) die Ereignisse der ersten Relevanzklasse in wenigstens zwei Validitätsklassen zu sortieren, umfassend eine erste Validitätsklasse mit bei einer Cheyne-Stokes-Atmung üblicherweise auftretenden Ereignissen (19, 29, 39, 49, 59) und eine zweite Validitätsklasse mit bei einer Cheyne-Stokes-Atmung üblicherweise nicht auftretenden Ereignissen, wobei die erste Validitätsklasse wenigstens die Ereignisse einer zentralen Apnoe und/oder einer zentralen Hypopnoe umfasst und / oder die zweite Validitätsklasse wenigstens die Ereignisse einer obstruktiven Apnoe und/oder einer obstruktiven Hypopnoe umfasst.

9. Beatmungsgerät (1) nach einem der drei vorhergehenden Ansprüche, wobei die Überwachungseinrichtung (5) dazu geeignet und ausgebildet ist, das Vorliegen einer Cheyne-Stokes-Atmung zu registrieren, wenn wenigstens drei Ereignisse (19, 29, 39, 49, 59) zeitlich aufeinander folgen und dabei wenigstens zwei der wenigstens drei Ereignisse der ersten Validitätsklasse entsprechen und maximal ein Ereignis der wenigstens drei Ereignisse der zweiten Validitätsklasse entspricht.

10. Beatmungsgerät (1) nach einem der vorhergehenden Ansprüche, wobei die Überwachungseinrichtung (5) dazu geeignet und ausgebildet ist, das Vorliegen einer Cheyne-Stokes-Atmung zu registrieren, wenn eine erste und eine zweite und eine dritte Periodenlänge (18) jeweils wenigstens 35 Sekunden und maximal 95 Sekunden betragen.

11. Beatmungsgerät (1) nach einem der vorhergehenden Ansprüche, wobei die Überwachungseinrichtung (5) dazu geeignet und ausgebildet ist, das Vorliegen einer Cheyne-Stokes-Atmung zu registrieren, wenn eine zeitliche Dauer jeweils eines der für die Ereignisanalyse (8) berücksichtigten Ereignisse (19, 29, 39, 49, 59) maximal 80% und insbesondere maximal 70% der Periodenlänge (18) beträgt.

12. Beatmungsgerät (1) nach einem der vorhergehenden Ansprüche, wobei die Überwachungseinrichtung (5) dazu geeignet und ausgebildet ist, wenigstens ein Flowsignal und/oder wenigstens ein Drucksignal von wenigstens einer zugeordneten Sensoreinrichtung (35) zur Erfassung wenigstens einer Floweigenschaft und/oder wenigstens einer Druckeigenschaft des Luftstroms für die Beatmung zu überwachen und anhand wenigstens eines Algorithmus auszuwerten, um ein Ereignis (19, 29, 39, 49, 59) zu typisieren.

13. Beatmungsgerät (1) nach einem der vorhergehenden Ansprüche, wobei die Überwachungseinrichtung (5) dazu geeignet und ausgebildet ist, bei einer registrierten Cheyne-Stokes-Atmung einen entsprechenden Hinweis an einen Benutzer auszugeben und/oder diese als entsprechende Atemstörung in einer Beatmungsstatistik zu berücksichtigen.

## Claims

1. A respiration device (1) having at least one respiration.unit (2) for generating an airflow for respiration and having at least one monitoring unit (5) for detecting at least one respiration parameter and for classifying events in the respiration on the basis of monitoring of the respiration parameter, wherein the monitoring unit (5) is able and designed to carry out at least one event analysis (8) to recognize an occurrence of chronologically successive events which is characteristic for Cheyne-Stokes respiration and for this purpose to ascertain period lengths (19, 29, 39, 49, 59) between the chronologically successive events and to compare the ascertained period lengths (18) at least partially to one another and **characterized in that** the ascertained period lengths (18) register the presence of Cheyne-Stokes respiration when the compared period lengths (18) deviate by less than 40% from one another.

2. The respiration device (1) as claimed in claim 1, wherein the monitoring unit (5) is able and designed to carry out at least one event analysis (8) for recognizing an occurrence of at least three chronologically successive events (19, 29, 39, 49, 59) which is characteristic for Cheyne-Stokes respiration and for this purpose to ascertain at least one first period length (18) between a first event and a second event and one second period length between a second event and a third event and to at least partially compare them to one another and to register the presence of Cheyne-Stokes respiration when the first period length (18) and the second period length (18) deviate from one another by less than 40%.

3. The respiration device (1) as claimed in any one of the preceding claims, wherein the monitoring unit (5) is able and designed to carry out at least one event analysis (8) for recognizing an occurrence of at least four chronologically successive events (19, 29, 39, 49, 59) which is characteristic for Cheyne-Stokes respiration and for this purpose to ascertain at least one first period length (18) between a first event and a second event and one second period length (18) between a second event and a third event and one third period length (18) between a third event and a fourth event and to at least partially compare them to one another and to register the presence of Cheyne-Stokes respiration when the first period length (18) and the second period length (18) and also the second period length (18) and the third period length (18) each deviate by less than 40% from one another.

4. The respiration device (1) as claimed in the preceding claim, wherein the monitoring unit (5) is able and designed to register the presence of Cheyne-Stokes respiration when the first period length (18) and the second period length (18) and also the second period length (18) and the third period length (18) each deviate by less than 35% and in particular by less than 30% from one another.

5. The respiration device (1) as claimed in any one of the preceding claims, wherein the period lengths (18) extend between a chronological middle of one event and a chronological middle of a following event.

6. The respiration device (1) as claimed in any one of the preceding claims, wherein the monitoring unit (5) is able and designed to sort the events into at least two relevance classes, comprising at least one first relevance class having events (19, 29, 39, 49, 59) which are relevant for Cheyne-Stokes respiration and at least one second relevance class having events which are irrelevant for Cheyne-Stokes respiration.

7. The respiration device (1) as claimed in the preceding claim, wherein the first relevance class comprises at least the events (19, 29, 39, 49, 59) of a hyperventilation and/or a hypoventilation and/or a hypopnea and/or an apnea and/or the second relevance class comprises at least the events of inspiration, expiration, snoring, swallowing, speaking, nose blowing, and/or coughing, and/or a respiration interruption and/or a leak.

8. The respiration device (1) as claimed in claim 6 or 7, wherein the monitoring unit (5) is able and designed to sort the events of the first relevance class into at least two validity classes for the event analysis (8), comprising a first validity class having events (19, 29, 39, 49, 59) which typically occur in the case of Cheyne-Stokes respiration and a second validity class having events which typically do not occur in the case of Cheyne-Stokes respiration, wherein the first validity class comprises at least the events of a central apnea and/or a central hypopnea and/or the second validity class comprises at least the events of an obstructive apnea and/or an obstructive hypopnea.

9. The respiration device (1) as claimed in any one of the three preceding claims, wherein the monitoring unit (5) is able and designed to register the presence of Cheyne-Stokes respiration when at least three events (19, 29, 39, 49, 59) occur in chronological succession and in this case at least two of the at least three events correspond to the first validity class and at most one event of the at least three events corresponds to the second validity class.

10. The respiration device (1) as claimed in any one of the preceding claims, wherein the monitoring unit (5) is able and designed to register the presence of Cheyne-Stokes respiration when a first and a second and a third period length are each at least 35 seconds and at most 95 seconds.

11. The respiration device (1) as claimed in any one of the preceding claims, wherein the monitoring unit (5) is able and designed to register the presence of Cheyne-Stokes respiration when a chronological duration of each one of the events (19, 29, 39, 49, 59) taken into consideration for the event analysis (8) is at most 80% and in particular at most 70% of the period length (18).

12. The respiration device (1) as claimed in any one of the preceding claims, wherein the monitoring unit (5) is able and designed to monitor at least one flow signal and/or at least one pressure signal from at least one associated sensor unit (35) to detect at least one flow property and/or at least one pressure property of the airflow for the respiration and to analyze it on the basis of at least one algorithm, in order to classify an event (19, 29, 39, 49, 59).

13. The respiration device (1) as claimed in any one of the preceding claims, wherein the monitoring unit (5) is able and designed to output a corresponding notification to a user in the event of registered Cheyne-Stokes respiration and/or to consider it as a corresponding respiratory disturbance in a respiration statistic.

## Revendications

1. Ventilateur (1) avec au moins un dispositif de ventilation (2) pour la génération d'un flux d'air destiné à la ventilation et avec au moins un dispositif de monitorage (5) pour l'enregistrement d'au moins un paramètre de ventilation et pour le typage d'événements (19, 29, 39, 49, 59) respiratoires à l'aide d'un monitorage du paramètre de ventilation, le dispositif de monitorage (5) étant approprié et conçu pour réaliser au moins une analyse d'événement (8) pour la détection d'événements consécutifs se succédant dans le temps et caractéristiques d'un épisode de respiration de Cheyne-Stokes et à cet effet pour déterminer des durées de périodes (18) entre les événements consécutifs se succédant dans le temps (19, 29, 39, 49, 59) et pour comparer entre elles les durées de périodes déterminées (18) et **caractérisé en ce que** les durées de périodes déterminées (18) sont enregistrées comme étant des épisodes de respiration de Cheyne-Stokes lorsque les durées de périodes comparées (18) s'écartent de moins de 40 % les unes des autres.

2. Ventilateur (1) selon la revendication 1 dont le dispositif de monitorage (5) est approprié et conçu pour réaliser au moins une analyse d'événement (8) pour la détection d'au moins trois événements consécutifs (19, 29, 39, 49, 59) caractéristiques d'un épisode de respiration de Cheyne-Stokes et à cet effet pour déterminer et comparer ente elles au moins une première durée de période (18) entre un premier événement et un deuxième et une deuxième durée de période (18) entre un deuxième événement et un troisième événement et les enregistrer comme étant des épisodes de respiration de Cheyne-Stokes lorsque la première durée de période (18) et la deuxième durée de période (18) s'écartent de moins de 40 % les unes des autres.

3. Ventilateur (1) selon l'une des revendications précédentes dont le dispositif de monitorage (5) est approprié et conçu pour réaliser au moins une analyse d'événement (8) pour la détection d'au moins quatre événements consécutifs (19, 29, 39, 49, 59) caractéristiques d'un épisode de respiration de Cheyne-Stokes et à cet effet pour déterminer et comparer entre elles au moins une première durée de période (18) entre un premier événement et un deuxième et une deuxième durée de période (18) et entre un deuxième événement et un troisième événement et une troisième durée de période (18) et entre un troisième événement et un quatrième événement et établir une respiration de Cheyne-Stokes lorsque la première durée de période (18) et la deuxième durée de période (18) ainsi que la deuxième durée de période (18) et la troisième durée de période (18) s'écartent chacune de moins de 40 % les unes des autres.

4. Ventilateur (1) selon l'une des revendications précédentes dont le dispositif de monitorage (5) est approprié et conçu pour établir la présence d'une respiration de Cheyne-Stokes lorsque la première durée de période (18) et la deuxième durée de période (18) et la deuxième duré de période (18) et la troisième durée de période (18) s'écartent chacune de moins de 35 % et en particulier de moins de 30 % les unes des autres.

5. Ventilateur (1) selon l'une des revendications précédentes, les durées de périodes (18) s'étalant entre un milieu temporel d'un événement et un milieu temporel d'un événement consécutif.

6. Ventilateur (1) selon l'une des revendications précédentes dont le dispositif de monitorage (5) est approprié et conçu pour trier les événements selon au moins deux classes de pertinences, comprenant au moins une première classe de pertinence avec des événements pertinents pour une respiration Cheyne-Stokes (19, 29, 39, 49, 59) et au moins une deuxième classe de pertinence avec des événements non pertinents pour une respiration Cheyne-Stokes.

7. Ventilateur (1) selon la revendication précédente avec lequel la première classe de pertinence comprend au moins les événements (19, 29, 39, 49, 59) d'une hyperventilation et/ou d'une hypoventilation et/ou d'une hypopnée et/ou d'une apnée et/ou la deuxième classe de pertinence comprend au moins les événements de respiration, d'expiration, de ronflement, de déglutition, de parole, de mouchage et/ou de toux et/ou une interruption de la ventilation et/ou une fuite.

8. Ventilateur (1) selon la revendication 6 ou 7 dont le dispositif de monitorage (5) est approprié et conçu pour l'analyse des événements (8) trier les événements de la première classe de pertinence selon au moins deux classes de validité, comprenant au moins une première classe de validité avec des événements survenant habituellement dans une respiration Cheyne-Stokes (19, 29, 39, 49, 59) et une deuxième classe de validité avec des événements ne survenant pas habituellement dans une respiration Cheyne-Stokes, la première classe de validité comprenant au moins les événements d'une hypopnée centrale et/ou la deuxième classe de validité comprenant au moins les événements d'une apnée obstructive et/ou d'une hypopnée obstructive.

9. Ventilateur (1) selon l'une des trois revendications précédentes dont le dispositif de monitorage (5) est approprié et conçu pour établir la présence d'une respiration de Cheyne-Stokes lorsqu'au moins trois événements (19, 29, 39, 49, 59) consécutifs et dont au moins deux des trois événements correspondent à la première classe de validité et au maximum un événement d'au moins trois événements correspond à la deuxième classe de validité.

10. Ventilateur (1) selon l'une des trois revendications précédentes dont le dispositif de monitorage (5) est approprié et conçu pour établir la présence d'une respiration de Cheyne-Stokes si une première, une deuxième et une troisième durée de période (18) durent chacune au moins 35 secondes et maximum 95 secondes.

11. Ventilateur (1) selon l'une des trois revendications précédentes dont le dispositif de monitorage (5) est approprié et conçu pour établir la présence d'une respiration de Cheyne-Stokes si une durée de période dure systématiquement maximum 80 % et en particulier maximum 70 % de la durée de période (18) pour les événements (19, 29, 39, 49, 59) à prendre en considération pour l'analyse des événements (8).

12. Ventilateur (1) selon l'une des trois revendications précédentes dont le dispositif de monitorage (5) est approprié et conçu pour surveiller au moins un signal de débit et/ou au moins un signal de pression délivré par au moins un dispositif de capteurs (35) associé pour enregistrer au moins une caractéristique de débit et/ou au moins un caractéristique de pression du flux d'air de la ventilation et pour procéder au typage d'un événement (19, 29, 39, 49, 59) à l'aide d'au moins algorithme.

13. Ventilateur (1) selon l'une des trois revendications précédentes dont le dispositif de monitorage (5) est approprié et conçu pour fournir à l'utilisateur, en cas de respiration Cheyne-Stokes établie, une information correspondante et/ou pour prendre cette respiration en considération dans une statistique de ventilation comme étant une défaillance respiratoire.
